# FASCICULE DE BREVET EUROPEEN

(11) **EP 1 377 265 B1**
(45) Date de publication et mention de la délivrance du brevet: **03.06.2009**
(21) Numéro de dépôt: 02757751.9
(22) Date de dépôt: 02.04.2002
(51) Int. Cl.: C09B 44/16, A61Q 5/10, A61Q 5/06, A61K 8/49

(54) **COMPOSITION TINCTORIALE POUR LA TEINTURE DES FIBRES KERATINIQUES COMPRENANT UN COLORANT AZOIQUE CATIONIQUE**
FÄRBEZUSAMMENSETZUNG ZUM FÄRBEN VON KERATINÖSEN FASERN MIT EINEM KATIONISCHEN AZO-FARBSTOFF
DYEING COMPOSITION FOR DYEING KERATINOUS FIBRES COMPRISING A CATIONIC AZO-DYE

(30) Priorité: 02.04.2001 FR 0104470
(43) Date de publication de la demande: 07.01.2004
(73) Titulaire: L'ORÉAL, 75008 Paris (FR)
(72) Inventeur: VIDAL, Laurent, F-75013 Paris (FR); FADLI, Aziz, F-77500 Chelles (FR)
(74) Mandataire: Wattremez, Catherine
(86) Numéro de dépôt international: PCT/FR2002/001139
(87) Numéro de publication internationale: WO 2002/078660

(56) Documents cités:
- EP-A- 0 850 636
- EP-A- 0 852 135
- WO-A-95/01772

## Description

L'invention a pour objet une nouvelle composition tinctoriale pour la teinture des fibres kératiniques, en particulier des cheveux humains, contenant un colorant azoïque cationique particulier, ainsi que le procédé de teinture des fibres kératiniques mettant en oeuvre une telle composition. L'invention a aussi pour objet des colorants cationiques azoïques nouveaux.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

Ce procédé de coloration d'oxydation consiste à appliquer sur le fibres kératiniques, des bases d'oxydation ou un mélange de bases d'oxydation et de coupleurs avec un agent oxydant, par exemple de l'eau oxygénée, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliquées à pH basique, il permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est à dire de la rendre plus visible.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer les fibres.

Il est connu par exemple d'utiliser des colorants directs du type nitrés benzèniques, anthraquinoniques, nitropyridiniques, des colorants du type azoïques, xanthéniques, acridiniques aziniques ou des colorants triarylméthane.

Les colorations qui en résultent sont des colorations particulièrement chromatique qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux. En outre, leur sensibilité à la lumière est dépendante de leur répartition uniforme ou en agrégats dans la fibre kératinique.

Il est connu d'utiliser des colorants directs en combinaison avec des agents oxydants. Cependant, les colorants directs sont généralement sensibles à l'action des agents oxydants tels que l'eau oxygénée, et les agents réducteurs tels que le bisulfite de sodium, ce qui les rendent généralement difficilement utilisables dans les compositions de teinture directe éclaircissantes à base d'eau oxygénée et à base d'un agent alcalinisant ou dans des compositions de teinture d'oxydation en association avec des précurseurs du type bases d'oxydation ou coupleurs.

Par exemple, il a été proposé dans les demandes de brevets FR-1 584 965 et JP-062 711 435 de teindre les cheveux avec des compositions de teinture à base de colorants directs nitrés et/ou de colorants dispersés azoïques et d'eau oxygénée ammoniacale en appliquant sur les cheveux un mélange desdits colorants et dudit oxydant, réalisé juste avant l'emploi. Mais les colorations obtenues se sont révélées insuffisamment tenaces et disparaissent aux shampooings en laissant apparaître l'éclaircissement de la fibre capillaire. Une telle coloration devient inesthétique en évoluant au cours du temps.

On a également proposé dans les demandes de brevets JP-53 95693 et JP-55 022638 de teindre les cheveux avec des compositions à base de colorants directs cationiques de type oxazine et d'eau oxygénée ammoniacale, en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pause des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.

Plus récemment, la demande de brevet FR 2 741 798 a décrit des compositions tinctoriales contenant des colorants directs comportant au moins un atome d'azote quaternisé du type azoïque ou azométhine, lesdites compositions étant à mélanger extemporanément à pH basique à une composition oxydante. Ces compositions permettent d'obtenir des colorations avec des reflets homogènes, tenaces et brillants. Cependant, elles ne permettent pas de teindre les fibres kératiniques avec autant de puissance qu'avec des compostions de coloration d'oxydation.

Il existe donc un réel besoin de rechercher des colorants directs chromatiques qui permettent de teindre les fibres kératiniques aussi puissamment que les colorants d'oxydation, qui soient aussi stables qu'eux à la lumière, soient également résistants aux intempéries, aux lavages et à la transpiration, et en outre, suffisamment stables en présence d'agents oxydants et réducteurs pour pouvoir obtenir simultanément un éclaircissement de la fibre soit par utilisation de compositions directes éclaircissantes les contenant, soit par l'utilisation de compositions de coloration d'oxydation les contenant. Il existe aussi un réel besoin de rechercher des colorants directs qui permettent de teindre les fibres kératiniques pour obtenir une gamme très large de couleurs, en particulier très chromatiques, sans oublier les nuances dites « fondamentale » comme les noirs et les marrons.

Ces buts sont atteints avec la présente invention qui a pour objet une composition pour la teinture des fibres kératiniques et en particulier des fibres kératiniques humaines telles que les cheveux, comprenant au moins un colorant azoïque cationique de formule (1) suivante :

**W₁-N=N-W₂-W₃**

dans laquelle
- W₁ représente un hétérocycle aromatique cationique à 5 chaînons de formule(II) suivante 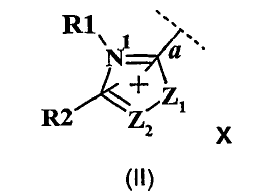
- W₂ représente un groupe divalent aromatique carboné ou pyridinique de formules (IV) ou (V) suivantes 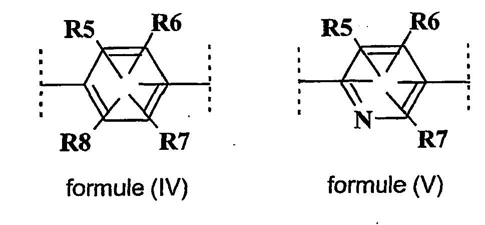
- W₃ représente un radical hétéroaromatique azoté à 5 ou 6 chaînons relié à W₂ par l'atome d'azote du cycle du radical hétéroaromatique, le radical héréroaromatique étant choisi parmi les radicaux pyrazolyle, pyrrolyle, imidazolyle, triazolyle, thiadiazolyle, pyridazinyle, pyrazinyle, chacun de ces hétéroaromatiques pouvant être éventuellement substitué par un ou plusieurs radicaux choisi parmi un atome d'hydrogène ; un radical alkyle en C₁-C₆, éventuellement substitué par un ou plusieurs radicaux hydroxy, alcoxy en C₁-C₄, (poly)-hydroxyalcoxy, amino, (di)alkylamino en C₁-C₄, (poly)hydroxyalkylamino en C₂-C₄, carboxy, sulfonyle, alcoxycarbonyle, thioether en C₁-C₄ ; un radical phényle éventuellement substitué par un ou plusieurs radicaux choisis parmi les radicaux alcoxy en C₁-C₂, amino, (di)-alkylamino en C₁-C₂, carboxy, sulfonyle, alkyle en C₁-C₄, halogène, thioéther en C₁-C₂,
- Z₁ représente un atome d'oxygène, de soufre ou un radical NR₄,
- Z₂ représente un atome d'azote ou un radical CR₃,
- R₁ et R₄ représentent, indépendamment l'un de l'autre, un radical alkyle en C₁-C₈, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂ , un carboxy, un sulfonique ; un radical phényle éventuellement substitué,
- R₂ et R₃ représentent, indépendamment l'un de l'autre, un atome d'hydrogène ; un radical alkyle en C₁-C₆, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy, un sulfonique; un radical phényle éventuellement substitué ; un radical carboxy ; un radical sulfonylamino ;
- R₅, R₆, R₇ et R₈ représentent, ensemble ou indépendamment l'un de l'autre un atome d'hydrogène ; un atome de chlore ; un atome de brome ; une chaîne hydrocarbonée en C₁-C₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogènes ; R₅, R₆, R₇ et R₈ ne comportant pas de liaison peroxyde, ni de radicaux diazo ou nitroso,
- X représente un anion organique ou minéral.

Dans le cadre de la présente invention, on entend par le terme "alkyle", sauf indication contraire, un radical alkyle comprenant de 1 à 10 atomes de carbone, de préférence de 1 à 6 atomes de carbone, pouvant être linéaire ou ramifié. Le terme alcoxy signifie alkyl-O-, le terme alkyle ayant la signification précédente.

Selon l'invention, lorsqu'il est indiqué qu'un ou plusieurs des atomes de carbone de la chaîne hydrocarbonée définie pour les radicaux R₅ à R₈ peuvent être remplacés par un atome d'oxygène, d'azote ou de soufre ou par un groupement SO₂, et/ou que ces chaines hydrocarbonées sont insaturées, cela signifie que l'on peut, à titre d'exemple, faire les transformations suivantes :

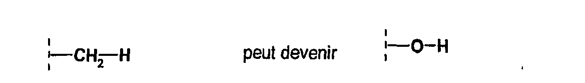

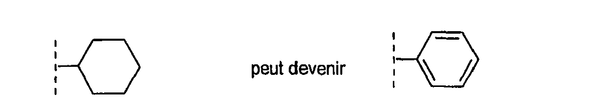

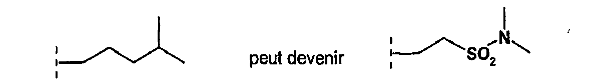

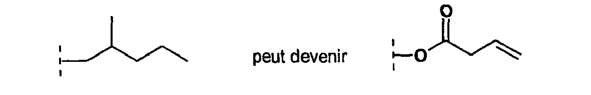

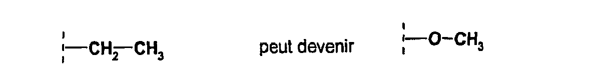

En particulier, on entend par "chaîne hydrocarbonée ramifiée", une chaîne pouvant former un ou plusieurs cycles carbonés comportant de 3 à 6 chaînons. On entend par chaîne hydrocarbonée insaturée, une chaîne pouvant comporter une ou plusieurs liaisons doubles et/ou une ou plusieurs liaisons triples, cette chaîne hydrocarbonée pouvant conduire à des groupements aromatiques.

Selon la présente invention, R₁ et R₄ représentent préférentiellement un radical alkyle en C₁-C₄ éventuellement substitué par un ou plusieurs radicaux choisis parmi les radicaux hydroxy, alcoxy en C₁-C₂, amino, (di)alkylamino en C₁-C₂, carboxy, ou sulfonique. Selon un mode de réalisation préféré, R₁ et R₄ représentent un radical méthyle, éthyle, 2-hydroxyéthyle, 1-carboxyméthyle, 2- carboxyéthyle, 2-sulfonyléthyle.

R₂ et R₃ représentent de préférence un atome d'hydrogène, un radical alkyle en C₁-C₄ éventuellement substitué par un ou plusieurs radicaux choisis parmi les radicaux hydroxy, amino, (di)alkylamino en C₁-C₂ , carboxy, un phényle. Selon un mode de réalisation particulier, R₂ et R₃ représentent préférentiellement un atome d'hydrogène, un radical méthyle, éthyle, 2-hydroxyéthyle, carboxy, 1-carboxyméthyle, 2- carboxyéthyle, 2-sulfonyléthyle.

R₅, R₆, R₇ et R₈ sont de préférence choisis parmi un atome d'hydrogène, un radical méthyle, éthyle, isopropyle, méthoxyméthyle, hydroxyméthyle, 1-carboxyméthyle, 1-aminométhyl, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle, méthoxy, éthoxy, 3-hydroxyéthyloxy, 3-aminoéthyloxy. Selon un mode de réalisation préféré, R₅, R₆, R₇ et R₈ sont choisis parmi un atome d'hydrogène, un radical méthyle, hydroxyméthyle, 2-hydroxyéthyle, 1,2-dihydroxyéthyle, méthoxy, 2-hydroxyéthoxy, plus préférentiellement un atome d'hydrogène, un radical méthyle, un radical méthoxy.

Selon l'invention, W3 est de préférence choisi parmi les radicaux pyrazolyle, imidazolyle, triazolyle, pyrrolyle.

Dans la formule (1), Z1 est de préférence NR4 et Z2 est de préférence CR3. W2 est de préférence un radical phénylène. W3 est de préférence un radical pyrazolique

Selon un mode de réalisation particulièrement préféré, Z1 est NR4, Z2 est CR3, et R1 est un radical alkyle, et R2 et R3 sont l'hydrogène.

L'anion minéral ou organique X peut être choisi parmi un halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que méthylsulfonate ; un arylsulfonate substitué ou non substitué par un radical alkyle en C₁-C₄ tel que par exemple un 4-toluylsulfonate

La concentration en colorant cationique azoïque de formule (1) peut varier entre 0,001 et 5% en poids environ par rapport au poids total de la composition tinctoriale, et de préférence entre environ 0,05 et 2%.

La composition de l'invention peut de plus comprendre un agent oxydant. Cet agent oxydant peut être n'importe quel agent oxydant utilisé de façon classique pour la décoloration des fibres kératiniques. L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition selon l'invention peut de plus comprendre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N-N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N-bis-(β-hydroxyéthyl) N,N'-bis-(4-aminophényl) tétraméthylènediamine, la N,N-bis-(4-méthy(-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut contenir de plus un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méfihyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β**-**hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β**-**hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont en général présents en quantité comprise entre 0,001 et 10 % en poids environ du poids total de la composition tinctoriale et plus préférentiellement de 0,005 à 6 %. La ou les bases d'oxydation sont présentes en quantité de préférence comprise entre 0,001 à 10 % en poids environ du poids total de la composition tinctoriale, et plus préférentiellement de 0,005 à 6 %.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir des colorants directs différents de ceux de formule (I), ces colorants pouvant notamment être choisis parmi les colorants nitrés de la série benzénique, les colorants directs cationiques, les colorants directs azoïques, les colorants directs méthiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de foi-mule (III) suivante : 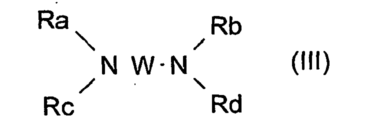 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe qui comprend l'application d'une composition tinctoriale contenant un colorant de formule (I) telle que définie précédemment sur les fibres kératiniques. Après un temps de pause, les fibres kératiniques sont rincées laissant apparaître des fibres colorées.

L'application sur les fibres de la composition tinctoriale contenant le colorant cationique azoïque de formule (I) peut être mise en oeuvre en présence d'agent oxydant ce qui provoque la décoloration de la fibre. Cet agent oxydant peut être ajouté à la composition contenant le colorant cationique azoïque au moment de l'emploi ou directement sur la fibre kératinique. Selon un mode de réalisation particulier, la composition contenant le colorant cationique azoïque de formule (I) est exempte de base d'oxydation et de coupleur.

L'invention a aussi pour objet un procédé de teinture d'oxydation permanente qui comprend l'application sur les fibres d'une composition tinctoriale qui comprend un colorant de formule (I), au moins une base d'oxydation et optionnellement au moins un coupleur, en présence d'un agent oxydant.

La base d'oxydation, le coupleur et l'agent oxydant sont tels que définis précédemment.

Dans le cadre de la teinture d'oxydation permanente, il est aussi possible d'utiliser comme agent oxydant des enzymes parmi lesquelles on peut citer les peroxydases, les oxydo-réductases à 2 électrons telles que les uricases et les oxygénases à 4 électrons comme les laccases.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée sur les fibres simultanément ou séquentiellement à la composition tinctoriale.

Dans le cas de la teinture d'oxydation permanente ou de la teinture directe, la composition tinctoriale est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pause de 3 à 50 minutes environ, de préférence 5 à 30 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Enfin l'invention a également pour objet les colorants azoïques cationiques de formule (1) telle que définie précédemment. Ces composés peuvent être obtenus à partir des procédés de préparation décrits par exemple dans les documents EP 810824, GB 9619573, RO 106572, J.Chem. Res.., Synop. (1998), (10), 648-649, DE 19721619, US 5852179, Synth. Commun 1999, 29(13), 2271-2276.

Les exemples qui suivent servent à illustrer l'invention sans toutefois présenter un caractère limitatif.

### EXEMPLES

### EXEMPLES DE SYNTHESE

### Exemple n° 1 : préparation d'un composé de formule :

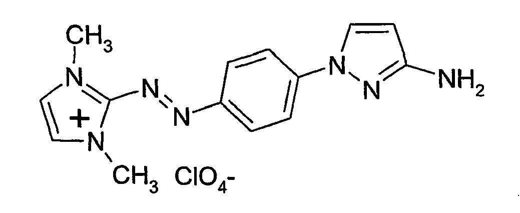

### Perchlorate de 2-[4(3-aminopyrazole-1-yl)-phenylazo)-1,3-dimethyl-3H-imidazol-1-ium.

Dans un ballon tout équipé , on charge 2,5 g (7,6 mmole) de perchlorate de 2-(4-methoxy-phenylazo)-1,3-dimethyl-3H-imidazol-1-ium et 50ml de 2-propanol . Le mélange est porté à 60°C. On ajoute 1,3 g (15,5 mmol) de 3-aminopyrazole. Ce mélange est porté sous agitation au reflux du solvant pendant 3 heures.
Sous agitation , on laisse revenir à température ambiante et un solide rouge est isolé par filtration, celui-ci est lavé 3 fois à l'éther diisopropylique.
Après séchage sous vide à 30°C, on récupère 1,65 g de Perchlorate de 2-[4(3 - aminopyrazole-1-yl)-phenylazo)-1,3-dimethyl-3H-imidazol-1-ium.sous forme de poudre rouge.

Les caractéristiques en absorption UV de ce produit sont les suivantes :
λₘₐₓ = 507 nm (HCOOH)

### Analyses :

Masse ESI+ : m/z = 382 [M⁺]
RMN 1H : (400MHz-DMSO) ppm :
4,36 ppm N-CH3
6,35 ppm 1 H pyrazole
7,95 ppm 2H aromatiques
8,11 ppm 2H imidazole
8,25 ppm 2H aromatiques

On obtient ainsi un colorant donnant une teinture rouge

### EXEMPLES DE TEINTURE

On a préparé les compositions tinctoriales suivantes

| Exemple | 3 |
|---|---|
| Colorant azoïque de l'exemple 1 | 5x10⁻⁴ mole |
| Polyéthylène glycol 8 OE | 12 g |
| Alcool benzylique | 10 g |
| Tampon Borate q.s.p | 100 g |
| pH | 9,2 |

Au moment de l'emploi, chaque composition est mélangée avec un poids égal d'eau oxygénée à 20 volumes (6% en poids).

Chaque mélange obtenu est appliqué sur des mèches de cheveux gris à 90 % de blancs, permanentés (BP) ou naturel (BN) (1 g de mèche pour 10 g de solution). Après 20 min de pose, les mèches sont rinçées, lavées avec un shampooing standard, rinçées à nouveau puis séchées.

Chaque mèche est évaluée avant et après la teinture dans le système L*a*b*, au moyen d'un spectrophotomètre CM 2002 MINOLTA ®, (Illuminant D65).

Dans l'espace L*a*b*, la clarté est indiquée par la valeur L* sur une échelle de 0 à 100 alors que les coordonnées chromatiques sont exprimées par a* et b* qui indiquent deux axes de couleur, a* l'axe rouge-vert et b* l'axe jaune-bleu.

Selon ce système, plus la valeur de L est élevée, plus la couleur est claire et peu intense. Inversement, plus la valeur de L est faible, plus la couleur est foncée ou très intense.

Les résultats de teinture suivants ont été obtenus.

| | **Cheveux naturels** | | | **Cheveux permanentés** | | |
|---|---|---|---|---|---|---|
| | **L*** | **a*** | **b*** | **L*** | **a*** | **b*** |
| **Exemple 1** | 21,5 | 5,3 | 3,15 | 18,6 | 5,4 | -1,1 |

## Revendications

1. Composition pour la teinture des fibres kératiniques et en particulier des fibres kératiniques humaines telles que les cheveux, comprenant au moins un colorant azoïque cationique de formule (1) suivante :
**W₁-N=N-W₂-W₃**
dans laquelle
- W₁ représente un hétérocycle aromatique cationique à 5 chaînons de formule(II) suivante 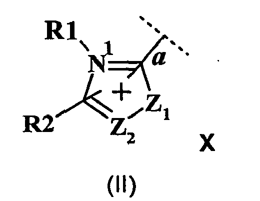
- W₂ représente un groupe divalent aromatique carboné ou pyridinique de formules (IV) ou (V) suivantes 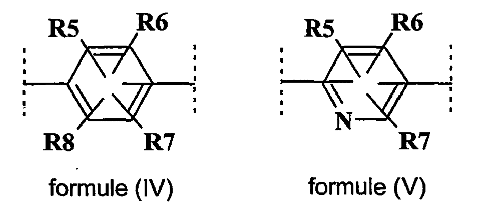
- W₃ représente un radical hétéroaromatique azoté à 5 ou 6 chaînons relié à W₂ par l'atome d'azote du cycle du radical hétéroaromatique, le radical héréroaromatique étant choisi parmi les radicaux pyrazolyle, pyrrolyle, imidazolyle, triazolyle, thiadiazolyle, pyridazinyle, pyrazinyle, chacun de ces hétéroaromatiques pouvant être éventuellement substitué par un ou plusieurs radicaux choisi parmi un atome d'hydrogène ; un radical alkyle en C₁-C₆, éventuellement substitué par un ou plusieurs radicaux hydroxy, alcoxy en C₁-C₄, (poly)-hydroxyalcoxy, amino, (di)alkylamino en C₁-C₄, (poly)hydroxyalkylamino en C₂-C₄, carboxy, sulfonyle, alcoxycarbonyle, thioether en C₁-C₄ ; un radical phényle éventuellement substitué par un ou plusieurs radicaux choisis parmi les radicaux alcoxy en C₁-C₂, amino, (di)-alkylamino en C₁-C₂, carboxy, sulfonyle, alkyle en C₁-C₄, halogène, thioéther en C₁-C₂,
- Z₁ représente un atome d'oxygène, de soufre ou un radical NR₄,
- Z₂ représente un atome d'azote ou un radical CR₃,
- R₁ et R₄ représentent, indépendamment l'un de l'autre, un radical alkyle en C₁-C₈, éventuellement substitué par un ou plusieurs radicaux choisis dans le groupe constitué par un hydroxy, un alcoxy en C₁-C₂, un radical (poly)-hydroxyalcoxy en C₂-C₄, un amino, un (di)alkylamino en C₁-C₂, un carboxy, un sulfonique ; un radical phényle éventuellement substitué,
- R₂ et R₃ représentent, indépendamment l'un de l'autre, un atome d'hydrogène ; un radical alkyle en C₁-C₆, éventuellement substitué par un ou plusieurs radicaux choisis dans le groupe constitué par un hydroxy, un alcoxy en C₁-C₂, un radical (poly)-hydroxyaicoxy en C₂-C₄, un amino, un (di)alkylamino en C₁-C₂, un carboxy, un sulfonique; un radical phényle éventuellement substitué ; un radical carboxy ; un radical sulfonylamino ;
- R₅, R₆, R₇ et R₈ représentent, ensemble ou indépendamment l'un de l'autre un atome d'hydrogène ; un atome de chlore ; un atome de brome ; une chaîne hydrocarbonée en C₁-C₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogènes ; R₅, R₆, R₇ et R₈ ne comportant pas de liaison peroxyde, ni de radicaux diazo ou nitroso,
- X représente un anion organique ou minéral.

2. Composition selon la revendication 1 dans laquelle R₁ et R₄ représentent un radical alkyle en C₁-C₄ éventuellement substitué par un ou plusieurs radicaux choisis parmi les radicaux hydroxy, alcoxy en C₁-C₂, amino, (di)alkylamino en C₁-C₂ , carboxy, ou sulfonique.

3. Composition selon la revendication 2 dans laquelle R₁ et R₄ représentent un radical méthyle, éthyle, 2-hydroxyéthyle, 1-carboxyméthyle, 2-carboxyéthyle, 2-sulfonyléthyle.

4. Composition selon l'une quelconque des revendications 1 à 3 dans laquelle R₂ et R₃ représentent de préférence un atome d'hydrogène, un radical alkyle en C₁-C₄ éventuellement substitué par un ou plusieurs radicaux choisis parmi les radicaux hydroxy, amino, (di)alkylamino en C₁-C₂ , carboxy, un phényle.

5. Composition selon la revendication 4 dans laquelle R₂ et R₃ représentent préférentiellement un atome d'hydrogène, un radical méthyle, éthyle, 2-hydroxyéthyle, carboxy, 1-carboxyméthyle, 2- carboxyéthyle, 2-sulfonyléthyle.

6. Composition selon l'une quelconque des revendications 1 à 5 dans laquelle R₅, R₅, R₇ et R₈ sont choisis parmi un atome d'hydrogène, un radical méthyle, éthyle, isopropyle, méthoxyméthyle, hydroxyméthyle, 1-carboxyméthyle, 1-aminométhyl, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle, méthoxy, éthoxy, 3-hydroxyéthyloxy, 3-aminoéthyloxy.

7. Composition selon la revendication 6 dans laquelle R₅, R₆, R₇ et R₈ sont choisis parmi un atome d'hydrogène ; un radical méthyle ; hydroxyméthyle ; 2-hydroxyéthyle ; 1,2-dihydroxyéthyle ; méthoxy; 2-hydroxyéthoxy, de préférence un atome d'hydrogène ; un radical méthyle ; un radical méthoxy.

8. Composition selon l'une quelconque des revendications 1 à 7 dans laquelle W3 est choisi parmi les radicaux pyrazolyle, imidazolyle, triazolyle, pyrrolyle.

9. Composition selon l'une quelconque des revendications 1 à 8 dans laquelle Z1 est NR4.

10. Composition selon l'une quelconque des revendications 1 à 8 dans laquelle Z2 est CR3.

11. Composition selon l'une quelconque des revendications précédentes dans laquelle W2 est un radical phénylène.

12. Composition selon l'une quelconque des revendications précédentes dans laquelle Z1 est NR4, Z2 est CR3, et R1 est un radical alkyle, et R2 et R3 sont l'hydrogène.

13. Composition selon l'une quelconque des revendications 1 à 13 dans laquelle W3 est un radical pyrazolique.

14. Composition selon l'une quelconque des revendications 1 à 13 comprenant un colorant de formule

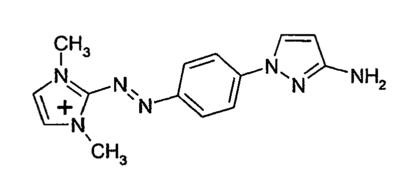

15. Composition selon l'une quelconque des revendications 1 à 14 comprenant de plus une base d'oxydation.

16. Composition selon la revendication 15 dans laquelle la base d'oxydation est choisie parmi les paraphéhylènediamines, les bis-phénylalkylènediamines, les para-aminophénols, les ortho-aminophénols, les bases hétérocycliques, et leurs sels d'addition avec un acide.

17. Composition selon l'une quelconque des revendications 15 ou 16 dans laquelle la ou les bases d'oxydation sont présentes en quantité comprise entre 0,001 et 10 %, de préférence entre 0,005 et 6 %.

18. Composition selon l'une quelconque des revendications 1 à 17 comprenant au moins un coupleur.

19. Composition selon la revendication 18 dans laquelle le coupleur est choisi parmi métaphénylènediamines, les méta-aminophénols, les métadiphénols, les coupleurs naphtaléniques et les coupleurs hétérocycliques et leur sel d'addition avec un acide.

20. Composition selon l'une quelconque des revendications 1 à 19 comprenant de plus un agent oxydant, de préférence le peroxyde d'hydrogène.

21. Procédé de teinture d'oxydation des fibres kératiniques et en particulier des fibres kératiniques humaines telles que les cheveux, **caractérisé en ce qu'**on applique sur les fibres au moins une composition tinctoriale telle que définie à l'une quelconque des revendications 1 à 19.

22. Procédé selon la revendication 21 dans lequel la composition tinctoriale contient un agent oxydant.

23. Procédé selon la revendication 21 dans lequel l'agent oxydant est mélangé au moment de l'emploi à la composition tinctoriale.

24. Procédé selon l'une quelconque des revendications 21 ou 22 dans lequel l'agent oxydant est appliqué sur les fibres sous forme de composition oxydante simultanément ou séquentiellement à la composition tinctoriale.

25. Procédé de teinture d'oxydation des fibres kératiniques et en particulier des fibres kératiniques humaines telles que les cheveux, **caractérisé en ce qu'**on applique sur les fibres au moins une composition tinctoriale telle que définie selon l'une quelconque des revendications 1 à 14, comprenant de plus au moins une base d'oxydation et optionnellement au moins un coupleur, en présence d'un agent oxydant.

26. Procédé selon la revendication 25 dans lequel l'agent oxydant est mélangé au moment de l'emploi à la composition tinctoriale.

27. Procédé selon la revendication 25 dans lequel l'agent oxydant est appliqué sur les fibres sous forme de composition oxydante simultanément ou séquentiellement à la composition tinctoriale.

28. Dispositif à plusieurs compartiments ou "kit" de teinture à plusieurs compartiments, dans lequel un premier compartiment contient une composition telle que définie à l'une quelconque des revendications 1 à 19 et un deuxième compartiment contient une composition oxydante.

29. Composés azoïques cationiques de formule (1) telle que définie selon l'une quelconque des revendications 1 à 14.

## Claims

1. Composition for dyeing keratinous fibres, and in particular human keratinous fibres such as the hair, comprising at least one cationic azo dye of the following formula (I):
**W₁-N=N-W₂-W₃**
in which
- W₁ represents a 5-membered cationic aromatic heterocycle of the following formula (II) 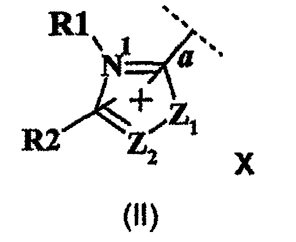
- W₂ represents a divalent carbonaceous aromatic or pyridine group of the following formulae (IV) or (V): 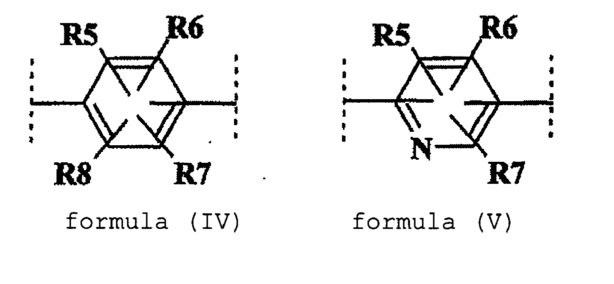
- W₃ represents a 5- or 6-membered nitrogen-containing heteroaromatic radical connected to W₂ by the nitrogen atom of the ring of the heteroaromatic radical, the heteroaromatic radical being chosen from the pyrazolyl, pyrrolyl, imidazolyl, triazolyl, thiadiazolyl, pyridazinyl and pyrazinyl radicals, it being possible for each of these heteroaromatic radicals to be optionally substituted by one or more radicals chosen from a hydrogen atom; a C₁-C₆ alkyl radical, optionally substituted by one or more hydroxyl, C₁-C₄ alkoxy, (poly)hydroxyalkoxy, amino, C₁-C₄ (di)alkylamino, C₂-C₄ (poly)hydroxyalkylamino, carboxyl, sulfonyl, alkoxycarbonyl, and C₁-C₄ thioether radicals; a phenyl radical optionally substituted by one or more radicals chosen from the C₁-C₂ alkoxy, amino, C₁-C₂ (di)alkylamino, carboxyl, sulfonyl, C₁-C₄ alkyl, halogen and C₁-C₂ thioether radicals,
- Z₁ represents an oxygen or sulfur atom or a radical NR₄,
- Z₂ represents a nitrogen atom or a radical CR₃,
- R₁ and R₄ represent, independently of one another, a C₁-C₈ alkyl radical, optionally substituted by one or more radicals chosen from the group comprising a hydroxyl, a C₁-C₂ alkoxy, a C₂-C₄ (poly)hydroxyalkoxy radical, an amino, a C₁-C₂ (di)alkylamino, a carboxyl, a sulfonic; an optionally substituted phenyl radical,
- R₂ and R₃ represent, independently of one another, a hydrogen atom; a C₁-C₆ alkyl radical, optionally substituted by one or more radicals chosen from the group comprising a hydroxyl, a C₁-C₂ alkoxy, a C₂-C₄ (poly)hydroxyalkoxy radical, an amino, a C₁-C₂ (di)alkylamino, a carboxyl, a sulfonic; an optionally substituted phenyl radical; a carboxyl radical; a sulfonylamino radical;
- R₅, R₆, R₇ and R₈ represent, together or independently of one another, a hydrogen atom; a chlorine atom; a bromine atom; a linear or branched C₁-C₆ hydrocarbonaceous chain which can form one or more 3- to 6-membered carbonaceous rings, and which can be saturated or unsaturated, of which one or more carbon atoms of the carbonaceous chain can be replaced by an oxygen, nitrogen or sulfur atom or by an SO₂ group, and the carbon atoms of which can be, independently of one another, substituted by one or more halogen atoms; R₅, R₆, R₇ and R₈ not comprising a peroxide bond or diazo or nitroso radicals,
- X represents an organic or inorganic anion.

2. Composition according to Claim 1, in which R₁ and R₄ represent a C₁-C₄ alkyl radical optionally substituted by one or more radicals chosen from the hydroxyl, C₁-C₂ alkoxy, amino, C₁-C₂ (di)alkylamino, carboxyl or sulfonic radicals.

3. Composition according to Claim 2, in which R₁ and R₄ represent a methyl, ethyl, 2-hydroxyethyl, 1-carboxymethyl, 2-carboxyethyl or 2-sulfonylethyl radical.

4. Composition according to any one of Claims 1 to 3, in which R₂ and R₃ preferably represent a hydrogen atom, a C₁-C₄ alkyl radical optionally substituted by one or more radicals chosen from hydroxyl, amino, C₁-C₂ (di)alkylamino and carboxyl radicals, and a phenyl.

5. Composition according to Claim 4, in which R₂ and R₃ preferably represent a hydrogen atom, a methyl, ethyl, 2-hydroxyethyl, carboxyl, 1-carboxymethyl, 2-carboxyethyl or 2-sulfonylethyl radical.

6. Composition according to any one of Claims 1 to 5, in which R₅, R₆, R₇ and R₈ are chosen from a hydrogen atom, a methyl, ethyl, isopropyl, methoxymethyl, hydroxymethyl, 1-carboxymethyl, 1-aminomethyl, 2-carboxyethyl, 2-hydroxyethyl, 3-hydroxypropyl, 1,2-dihydroxyethyl, 1-hydroxy-2-aminoethyl, 2-hydroxy-1-aminoethyl, methoxy, ethoxy, 3-hydroxyethyloxy or 3-aminoethyloxy radical.

7. Composition according to Claim 6, in which R₅, R₆, R₇ and R₈ are chosen from a hydrogen atom, a methyl, hydroxymethyl, 2-hydroxyethyl, 1,2-dihydroxyethyl, methoxy or 2-hydroxyethoxy radical, more preferably a hydrogen atom, a methyl radical or a methoxy radical.

8. Composition according to any one of Claims 1 to 7, in which W3 is chosen from the pyrazolyl, imidazolyl, triazolyl and pyrrolyl radicals.

9. Composition according to any one of Claims 1 to 8, in which Z1 is NR4.

10. Composition according to any one of Claims 1 to 8, in which Z2 is CR3.

11. Composition according to any one of the preceding claims, in which W2 is a phenylene radical.

12. Composition according to any one of the preceding claims, in which Z1 is NR4, Z2 is CR3, and R1 is an alkyl radical, and R2 and R3 are hydrogen.

13. Composition according to any one of Claims 1 to 12, in which W3 is a pyrazole radical.

14. Composition according to any one of Claims 1 to 13, containing a dye of formula

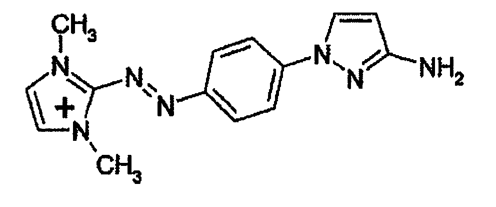

15. Composition according to any one of Claims 1 to 14, furthermore comprising an oxidation base.

16. Composition according to Claim 15, in which the oxidation base is chosen from para-phenylenediamines, bisphenylalkylenediamines, para-aminophenols, ortho-aminophenols, heterocyclic bases and their addition salts with an acid.

17. Composition according to either of Claims 15 and 16, in which the oxidation base(s) are present in a quantity between 0.001 and 10%, preferably between 0.005 and 6%.

18. Composition according to any one of Claims 1 to 17, comprising at least one coupler.

19. Composition according to Claim 18, in which the coupler is chosen from meta-phenylenediamines, meta-aminophenols, meta-diphenols, naphthalene couplers and heterocyclic couplers and their addition salt with an acid.

20. Composition according to any one of Claims 1 to 19, furthermore comprising an oxidizing agent, preferably hydrogen peroxide.

21. Process for the oxidation dyeing of keratinous fibres and in particular of human keratinous fibres such as the hair, **characterized in that** at least one dyeing composition as defined in any one of Claims 1 to 19 is applied to the fibres.

22. Process according to Claim 21, in which the dyeing composition contains an oxidizing agent.

23. Process according to Claim 21, in which the oxidizing agent is mixed at the time of use with the dyeing composition.

24. Process according to either of Claims 21 and 22, in which the oxidizing agent is applied to the fibres in the form of an oxidizing composition simultaneously with or sequentially to the dyeing composition.

25. Process for the oxidation dyeing of the keratinous fibres and in particular of human keratinous fibres such as the hair, **characterized in that** at least one dyeing composition as defined in any one of Claims 1 to 14, furthermore comprising at least one oxidation base and optionally at least one coupler, in the presence of an oxidizing agent, is applied to the fibres.

26. Process according to Claim 25, in which the oxidizing agent is mixed at the time of use with the dyeing composition.

27. Process according to Claim 25, in which the oxidizing agent is applied to the fibres in the form of an oxidizing composition simultaneously with or sequentially to the dyeing composition.

28. Multi-compartment device or multi-compartment dyeing "kit", in which a first compartment comprises a composition as defined in any one of Claims 1 to 19 and a second compartment comprises an oxidizing composition.

29. Cationic azo compound of formula (I) as defined according to any one of Claims 1 to 14.

## Patentansprüche

1. Zusammensetzung zum Färben von Keratinfasern und insbesondere menschlichen Keratinfasern, wie Haaren, die mindestens einen kationischen Azofarbstoff der folgenden Formel (I) enthält:
**W₁-N=N-W₂-W₃**
in der Formel:
- W₁ bedeutet einen 5-gliedrigen, kationischen aromatischen Heterocyclus der folgenden Formel (II): 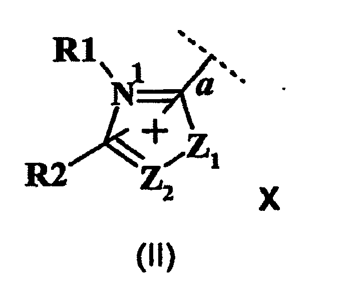
- W₂ bedeutet eine zweiwertige aromatische Kohlenstoffgruppe oder Pyridingruppe der folgenden Formeln (IV) oder (V): 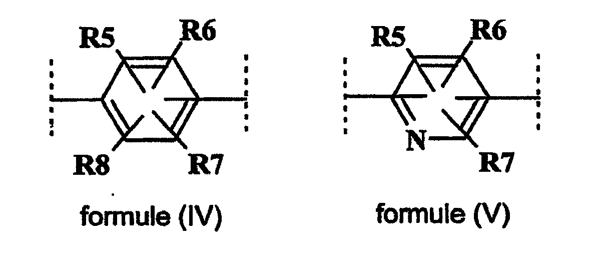
- W₃ bedeutet eine 5- oder 6-gliedrige, stickstoffhaltige heteroaromatische Gruppe, die über das Stickstoffatom des Rings der heteroaromatischen Gruppe an W₂ gebunden ist, wobei die heteroaromatische Gruppe unter den Gruppen Pyrazolyl, Pyrrolyl, Imidazolyl, Triazolyl, Thiadiazolyl, Pyridazinyl, Pyrazinyl ausgewählt ist und wobei jede dieser heteroaromatischen Gruppen gegebenenfalls mit einer oder mehreren Gruppen substituiert sein kann, die ausgewählt sind unter einem Wasserstoffatom; einer C₁₋₆-Alkylgruppe, die gegebenenfalls substituiert ist mit einer oder mehreren Gruppen Hydroxy, Alkoxy(C₁₋₄), (Poly)hydroxyalkoxy, Amino, (Di)alkylamino(C₁₋₄), (Poly)hydroxyalkylamino(C₂₋₄), Carboxy, Sulfonyl, Alkoxycarbonyl, Thioether(C₁₋₄); einer Phenylgruppe, die gegebenenfalls substituiert ist mit einer oder mehreren Gruppen, die unter den Gruppen Alkoxy(C₁₋₄), Amino, (Di)alkylamino(C₁₋₂), Carboxy, Sulfonyl, Alkyl(C₁₋₄), Halogen, Thioether(C₁₋₂) ausgewählt ist,
- Z₁ bedeutet ein Sauerstoffatom, ein Schwefelatom oder eine Gruppe NR₄,
- Z₂ bedeutet ein Stickstoffatom oder eine Gruppe CR₃,
- R₁ und R₄ bedeuten unabhängig voneinander eine C₁₋₈-Alkylgruppe, die gegebenenfalls mit einer oder mehreren Gruppen substituiert ist, die ausgewählt sind unter Hydroxy, Alkoxy(C₁₋₂), (Poly)hydroxyalkoxy(C₂₋₄), Amino, (Di)alkylamino(C₁₋₂), Carboxy, Sulfonyl; eine Phenylgruppe, die gegebenenfalls substituiert ist,
- R₂ und R₃ bedeuten unabhängig voneinander ein Wasserstoffatom; eine C₁₋₆-Alkylgruppe, die gegebenenfalls mit einer oder mehreren Gruppen substituiert ist, die ausgewählt sind unter Hydroxy, Alkoxy(C₁₋₂), (Poly)hydroxyalkoxy(C₂₋₄), Amino, (Di)alkylamino(C₁-₂), Carboxy, Sulfonyl; eine Phenylgruppe, die gegebenenfalls substituiert ist; eine Carboxygruppe; eine Sulfonylaminogruppe;
- R₅, R₆, R₇ und R₈ bedeuten gleichzeitig oder unabhängig voneinander ein Wasserstoffatom; ein Chloratom; ein Bromatom; eine geradkettige oder verzweigte C₁₋₆-Kohlenwasserstoffkette, die einen oder mehrere 3- bis 6-gliedrige Kohlenstoffringe bilden kann und die gesättigt oder ungesättigt vorliegen kann, bei der ein oder mehrere Kohlenstoffatome der Kohlenstoffkette durch ein Sauerstoffatom, Stickstoffatom oder Schwefelatom oder durch eine Gruppe SO₂ ersetzt sein können und bei der die Kohlenstoffatome unabhängig voneinander mit einem oder mehreren Halogenatomen substituiert sein können; wobei die Gruppen R₅, R₆, R₇ und R₈ weder eine Peroxidbindung noch eine Diazo- oder Nitrosogruppe enthalten;
- X bedeutet ein organisches oder anorganisches Anion.

2. Zusammensetzung nach Anspruch 1, wobei R₁ und R₄ eine C₁-₄-Alkylgruppe bedeuten, die gegebenenfalls mit einer oder mehreren Gruppen substituiert ist, die unter Hydroxy, Alkoxy(C₁₋₂), Amino, (Di)alkylamino(C₁-₂), Carboxy oder Sulfonyl ausgewählt sind.

3. Zusammensetzung nach Anspruch 2, wobei R₁ und R₄ Methyl, Ethyl, 2-Hydroxyethyl, 1-Carboxymethyl, 2-Carboxyethyl, 2-Sulfonylethyl bedeuten.

4. Zusammensetzung nach einem der Ansprüche 1 bis 3, wobei R₂ und R₃ vorzugsweise ein Wasserstoffatom, eine C₁₋₄-Alkylgruppe, die gegebenenfalls mit einer oder mehreren Gruppen substituiert ist, die unter Hydroxy, Amino, (Di)alkylamino(C₁-₂), Carboxy ausgewählt sind, eine Phenylgruppe bedeuten.

5. Zusammensetzung nach Anspruch 4, wobei R₂ und R₃ vorzugsweise ein Wasserstoffatom, Methyl, Ethyl, 2-Hydroxyethyl, Carboxy, 1-Carboxymethyl, 2-Carboxyethyl, 2-Sulfonylethyl bedeuten.

6. Zusammensetzung nach einem der Ansprüche 1 bis 5, wobei R₅, R₆, R₇ und R₈ unter einem Wasserstoffatom; Methyl, Ethyl, Isopropyl, Methoxymethyl, Hydroxymethyl, 1-Carboxymethyl, 1-Aminomethyl, 2-Carboxyethyl, 2-Hydroxyethyl, 3-Hydroxypropyl, 1,2-Dihydroxyethyl, 1-Hydroxy-2-aminoethyl, 2-Hydroxy-1-aminoethyl, Methoxy, Ethoxy, 3-Hydroxyethyloxy, 3-Aminoethyloxy ausgewählt sind.

7. Zusammensetzung nach Anspruch 6, wobei R₅, R₆, R₇ und R₈ unter einem Wasserstoffatom; Methyl; Hydroxymethyl; 2-Hydroxyethyl; 1,2-Dihydroxyethyl; Methoxy; 2-Hydroxyethyloxy und vorzugsweise einem Wasserstoffatom; Methyl; Methoxy ausgewählt sind.

8. Zusammensetzung nach einem der Ansprüche 1 bis 7, wobei W₃ unter Pyrazolyl, Imidazolyl, Triazolyl, Pyrrolyl ausgewählt ist.

9. Zusammensetzung nach einem der Ansprüche 1 bis 8, wobei Z₁ NR₄ bedeutet.

10. Zusammensetzung nach einem der Ansprüche 1 bis 8, wobei Z₂ CR₃ bedeutet.

11. Zusammensetzung nach einem der vorhergehenden Ansprüche, wobei W₂ eine Phenylgruppe ist.

12. Zusammensetzung nach einem der vorhergehenden Ansprüche, wobei Z₁ NR₄ bedeutet, Z₂ CR₃ ist, R₁ eine Alkylgruppe bedeutet und R₂ und R₃ Wasserstoff bedeuten.

13. Zusammensetzung nach einem der Ansprüche 1 bis 13, wobei W₃ eine Pyrazolgruppe ist.

14. Zusammensetzung nach einem der Ansprüche 1 bis 13, die einen Farbstoff der folgenden Formel enthält:

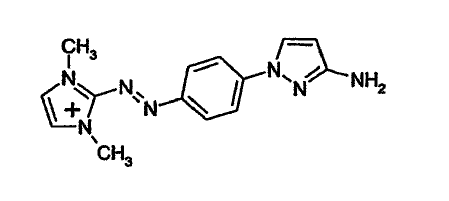

15. Zusammensetzung nach einem der Ansprüche 1 bis 14, die ferner eine Oxidationsbase enthält.

16. Zusammensetzung nach Anspruch 15, wobei die Oxidationsbase unter den *p-*Phenylendiaminen, Bisphenylalkylendiaminen, *p-*Aminophenolen, o-Aminophenolen, heterocyclischen Basen und deren Additionssalzen mit einer Säure ausgewählt ist.

17. Zusammensetzung nach einem der Ansprüche 15 oder 16, wobei die Oxidationsbase oder die Oxidationsbasen in einem Mengenanteil von 0,001 bis 10 % und vorzugsweise 0,005 bis 6 % enthalten sind.

18. Zusammensetzung nach einem der Ansprüche 1 bis 17, die mindestens einen Kuppler enthält.

19. Zusammensetzung nach Anspruch 18, wobei der Kuppler unter den m-Phenylendiaminen, m-Aminophenolen, m-Dihydroxybenzolen, Naphthalinkupplern und heterocyclischen Kupplern und deren Additionssalzen mit einer Säure ausgewählt ist.

20. Zusammensetzung nach einem der Ansprüche 1 bis 19, die ferner ein Oxidationsmittel und vorzugsweise Wasserstoffperoxid enthält.

21. Verfahren zum oxidativen Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 19 aufgebracht wird.

22. Verfahren nach Anspruch 21, wobei die Farbmittelzusammensetzung ein Oxidationsmittel enthält.

23. Verfahren nach Anspruch 21, wobei das Oxidationsmittel bei der Anwendung mit der Farbmittelzusammensetzung vermischt wird.

24. Verfahren nach einem der Ansprüche 21 oder 22, wobei das Oxidationsmittel in Form einer oxidierenden Zusammensetzung gleichzeitig mit oder nach der Farbmittelzusammensetzung auf die Fasern aufgebracht wird.

25. Verfahren für die oxidative Färbung von Keratinfasern und insbesondere menschlichen Keratinfasern, wie Haaren, **dadurch gekennzeichnet, dass** mindestens eine Farbmittelzusammensetzung nach einem der Ansprüche 1 bis 14, die ferner mindestens eine Oxidationsbase und gegebenenfalls mindestens einen Kuppler enthält, in Gegenwart eines Oxidationsmittels auf die Fasern aufgebracht wird.

26. Verfahren nach Anspruch 25, wobei das Oxidationsmittel bei der Anwendung mit der Farbmittelzusammensetzung vermischt wird.

27. Verfahren nach Anspruch 25, wobei das Oxidationsmittel in Form einer oxidierenden Zusammensetzung gleichzeitig mit oder nach der Farbmittelzusammensetzung auf die Fasern aufgetragen wird.

28. Vorrichtung mit mehreren Abteilungen oder "Kit" zum Färben mit mehreren Abteilungen, bei der eine erste Abteilung eine Zusammensetzung, wie sie in einem der Ansprüche 1 bis 19 definiert ist, und eine zweite Abteilung eine oxidierende Zusammensetzung enthält.

29. Kationische Azoverbindungen der Formel (I), wie sie in einem der Ansprüche 1 bis 14 definiert sind.
